# FASCICULE DE BREVET EUROPEEN

(11) **EP 0 897 717 B1**
(45) Date de publication et mention de la délivrance du brevet: **09.06.2004**
(21) Numéro de dépôt: 98480048.2
(22) Date de dépôt: 17.07.1998
(51) Int. Cl.: A61K 7/48

(54) **Composition cosmétique utile notamment pour le blanchiment de la peau et agent inhibiteur de la mélanogénèse comprenant une telle composition cosmétique**
Kosmetische Zusammensetzung zur Bleichung der Haut und Inhibitor der Melaninbildung, der eine solche Kosmetische Zusammensetzung enthält
Whitening skin cosmetic composition and melanogenic inhibitor agent containing such cosmetic composition

(30) Priorité: 31.07.1997 MC 2383
(43) Date de publication de la demande: 24.02.1999
(73) Titulaire: EXSYMOL S.A.M., MC-98000 Monte Carlo (MC); BABIZHAYEV, Marc, 74, Moscou, 127434 (RU)
(72) Inventeur: Seguin, Marie-Christine, 98000 Monaco (MC); Babizhayev, Mark A., 127434 Moscou (RU)
(74) Mandataire: Bonneau, Gérard

(56) Documents cités:
- PATENT ABSTRACTS OF JAPAN vol. 97, no. 4, 30 avril 1997 (1997-04-30) & JP 08 333235 A (KAO CORP.), 17 décembre 1996 (1996-12-17)
- PATENT ABSTRACTS OF JAPAN vol. 97, no. 4, 30 avril 1997 (1997-04-30) & JP 08 333229 A (KAO CORP.), 17 décembre 1996 (1996-12-17)
- PATENT ABSTRACTS OF JAPAN vol. 10, no. 235 (C-366), 14 août 1986 (1986-08-14) & JP 61 069707 A (SOGO YATSUKOU KK), 10 avril 1986 (1986-04-10)
- B. LEJCZAK ET AL.: "Phosphonic and phosphinic acid analogues of tyrosine and 3,4-dihydrophenylalanine (dopa) as potential antimelanotic agents" ANTICANCER DRUG DESIGN, vol. 5, no. 4, novembre 1990 (1990-11), pages 351-358, XP002115391

## Description

La présente invention a pour objet une composition cosmétique utile notamment pour le blanchiment de la peau, comprenant un composé de formule générale (I) décrite ci-dessous, en association avec tout excipient approprié ; la présente invention a également pour objet un agent inhibiteur de la mélanogénèse comprenant une telle composition cosmétique.

Les composés (I) répondent à la formule générale suivante : 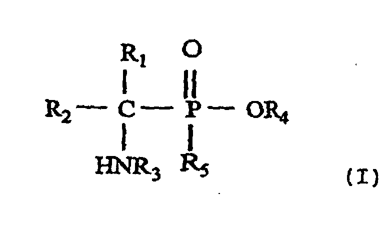 dans laquelle :
R₁ représente un atome d'hydrogène, un groupement (C₁-C₄) alkyle linéaire ou ramifié ou un groupement thiazoline,
R₂,R₃, et R₄ représentent chacun un atome d'hydrogène,
R₅ représente un atome d'hydrogène ou un groupement hydroxyle.

Depuis plusieurs années, on constate en cosmétique un intérêt croissant pour les produits dépigmentants : les taches de vieillesse (lentigo sénile), le masque de grossesse (chloasma), les taches de rousseur, les taches de pigmentations qui apparaissent sur les peaux matures après une exposition prolongée au soleil, sont mal supportés par ceux qui en sont atteints. Les phénomènes de mode privilégiant les peaux claires en Asie et en Afrique, représentent également des facteurs visant à promouvoir l'éclosion de produits dépigmentants.

La mélanine est un pigment synthétisé par les mélanocytes, cellules spécialisées situées au niveau de la couche basale de l'épiderme. Les hyperpigmentations sont souvent dues à une hyperactivité mélanocytaire, éventuellement accompagnée d'une augmentation du nombre de mélanocytes.
Au sein des mélanocytes, les réactions d'oxydation conduisant à la formation de mélanine sont principalement catalysées par la tyrosinase. Les pigments formés se répartissent uniformément à la surface de l'épiderme, sauf quant le mécanisme se dérègle : la mélanine s'accumule alors anarchiquement sur certains sites.

Les propriétés dépigmentantes de certaines substances sont connues depuis longtemps : dès 1936, Oettel observa la diminution de la pigmentation des poils de chats noirs auxquels de l'hydroquinone avait été administrée (Hemsworth B.N., J. Soc. Cosmet. Chem, 1973, 24,727-733).

Un certain nombre de substances ont montré une activité dépigmentante, mais certaines d'entre elles ne peuvent être utilisées en cosmétique à cause de leur toxicité (par exemple, les sels de mercure), de l'irritation cutanée qu'elles provoquent (mercaptoamines, produits oxydants tels que le peroxyde d'hydrogène) ou du fait de la lenteur excessive de leur action (acide ascorbique ou dérivés d'acide ascorbique).

D'autres composés tels que les dérivés phénoliques, les corticostéroïdes, les analogues de la vitamine A, certains extraits végétaux entrent dans la composition de nombreuses préparations dépigmentantes actuellement commercialisées.

Toutefois, ces composés présentent un certain nombre d'inconvénients : parmi les dérivés phénoliques, l'hydroquinone, qui est inhibitrice de la tyrosinase, éveille un nombre non négligeable de réactions secondaires (réactions inflammatoires, brûlures, picotements, dyschromie irréversible en confettis) ; les corticostéroïdes entraînent également de telles manifestations secondaires qu'ils sont inutilisables en cosmétique ; les analogues de la vitamine A (béta-carotène, canthaxantine) sont capables de bloquer l'activité mélanocytaires mais le problème posé par ces substances est d'obtenir une pénétration dans les cellules sans qu'elles soient métabolisées par les enzymes cellulaires.

Les extraits végétaux, qui contiennent, soit de l'arbutine (glucose hydroquinone), soit des flavonoïdes, ou encore des dérivés cinnamiques, sont également utilisés comme dépigmentants mais ont une action très lente.

De manière générale, le problème de ces dépigmentants de l'art antérieur est que deux mois minimum de traitement sont nécessaires pour voir apparaître les premiers résultats *in vivo*.

Pour notamment remédier à ces inconvénients, mais aussi se démarquer des compositions blanchissantes à base de dérivés aminophosphoniques, objets des abrégés des documents JP 08333235 et JP 61069707, un but de la présente invention est de proposer l'utilisation, pour le blanchiment de la peau, de composés dont la structure et le procédé de préparation sont connus, mais qui présentent une activité surprenante pour le blanchiment de la peau.

Un but de la présente invention est de proposer une composition cosmétique utile notamment pour le blanchiment de la peau, caractérisée en ce qu'elle contient, en association avec tout excipient approprié, au moins un composé de formule générale (I) : 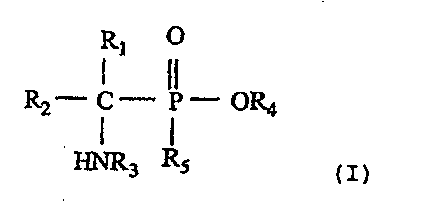 dans laquelle :
R₁ représente un atome d'hydrogène, un groupement (C₁-C₄) alkyle linéaire ou ramifié ou un groupement thiazoline,
R₂,R₃ et R₄ représentent chacun un atome d'hydrogène,
R₅ représente un atome d'hydrogène ou un groupement hydroxyle.

Avantageusement, le composé de formule (I) est tel que R₁ représente un groupement (C₁-C₄) alkyle, R₂ et R₃ représentent chacun un atome d' hydrogène et R₅ représente un atome d'hydrogène.

Suivant un mode de réalisation préféré de l'invention, lé composé de formule (I) est l'acide 1-amino-éthylphosphinique. Suivant un autre de mode de réalisation préféré de l'invention, le composé de formule (I) est l'acide 1-amino-3-méthylbutylphosphinique.

De préférence, la composition cosmétique selon l'invention comprend, outre le ou les composés de formule générale (I), des anti-inflammatoires, d'autres agents de blanchiment de la peau, et/ou des antioxydants.

Avantageusement, la teneur en composé de formule générale (I) est comprise entre 0,1 et 20 % en poids par rapport au poids total de la composition et de préférence entre 0,5 et 5 % en poids par rapport au poids total de la composition.

Suivant un mode de réalisation particulier, la composition selon l'invention comprend en outre un composé organo-silicé de formule générale (II) suivante : 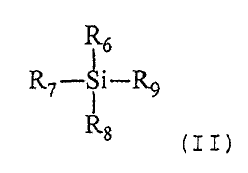 dans laquelle un au moins des radicaux R₆, R₇, R₈ ou R₉ représente un groupement hydroxyle ou un groupement alcoxy, acyloxy ou aryloxy, amine ou un atome d'halogène et au moins un autre desdits radicaux R₆, R₇, R₈ ou R₉ représente un groupement alkyle.

Suivant un mode de réalisation particulier de l'invention, la composition cosmétique comprend en outre un pseudo-dipeptide du type de ceux obtenus par couplage de l'histamine et d'un acide aminé.

De préférence, ce pseudo-dipeptide est la β-alanyl-histamine.

De préférence, l'excipient utilisé dans la composition cosmétique selon l'invention, est une micro-dispersion sans tensio-actif.

Avantageusement, la composition cosmétique selon l'invention se présente sous forme de liposomes, de microparticules ou de nanoparticules.

Suivant une variante avantageuse de l'invention, la composition cosmétique selon l'invention se présente sous la forme d'une crème, d'un lait, d'une lotion, ou d'un gel.

Un autre objet de l'invention est de proposer un agent inhibiteur de la mélanogénèse, caractérisé en ce qu'il comprend une composition cosmétique telle que décrite ci-dessus.

La présente invention se comprendra mieux au vu de la description et des exemples qui suivent, qui illustrent non limitativement l'invention.

Les composés de formule générale (I) sont disponibles dans le commerce ou peuvent être préparé suivant des méthodes décrites dans l'art antérieur, et notamment en suivant le protocole décrit dans J. Chem.Soc.Perkin.trans.I, 1984 par BAYLIS et al.

Les composés de formule (I) sont ceux pour lesquels R₁ représente un atome d' hydrogène, un groupement (C₁-C₄) alkyle linéaire ou ramifié ou un groupement thiazoline, R₂, R₃ et R₄ représentent chacun un atome d'hydrogène, et R₅ représente un atome d'hydrogène ou un groupement hydroxyle.

Parmi ces composés, les acides aminophosphiniques, c'est à dire les composés de formule (I) dans laquelle R₅ représente un atome d'hydrogène, sont particulièrement préférés.

Les composés selon l'invention sont des acides alpha-amino phosphoniques ou alpha-amino phosphiniques et présentent ainsi une structure proche de celle d'un acide alpha-aminé naturel.

Les composés entrant dans les compositions selon l'invention ont en eux-mêmes une action inhibitrice de la mélanogénèse. Les mécanismes de cette action ne sont pas exactement connus, et il est possible que l'activité constatée résulte de plusieurs mécanismes agissant simultanément et éventuellement de manière synergique.

Ainsi les composés selon l'invention peuvent être utiles pour le blanchiment de la peau parce qu'ils sont inhibiteurs de la tyrosinase, et/ou parce qu'ils agissent à d'autres niveaux.

Suivant un mode de réalisation préféré de l'invention, dans les compositions cosmétiques selon l'invention, on ajoute au composé de formule générale (I) au moins un autre composé utile pour le blanchiment de la peau. Avantageusement, le(s)dit(s) autre (s) composé(s) utile(s) pour le blanchiment de la peau comprennent au moins un inhibiteur de la tyrosinase.

De préférence, le(s) dit(s) autre(s) composé(s) utile(s) pour le blanchiment de la peau sont l'acide ascorbique et/ou ses dérivés et notamment ses sels de stéarate ou de palmitate ou encore l'acide ascorbique conjugué à un stabilisant, tel que notamment le composé commercialisé sous le nom de marque ASCORBOSILANE C par la Demanderesse.

Le(s) dit(s) autre (s) composé(s) utile(s) pour le blanchiment de la peau peuvent également être de l'acide kojique ou ses dérivés.

Suivant une variante avantageuse de l'invention, le(s)dit(s) autre(s) composé(s) utile(s) pour le blanchiment de la peau sont des extraits naturels de plantes, et plus particulièrement l'extrait de Mûrier blanc (*Morus alba*), le thé vert (*Théa sinensis*), la matricaria (*Chamomilla recutica*). Le thé vert est connu pour ses propriétés à inhiber le transport des mélanocytes matures vers les kératinocytes. C'est également un inhibiteur de la tyrosinase. La matricaria est un endothéline antagoniste, qui inhibe la mélanogénèse induite par les rayons ultraviolets.

Suivant un autre mode de réalisation particulier de l'invention, on ajoute dans les compositions cosmétiques selon l'invention au moins un anti-inflammatoire, notamment à vocation apaisante. Avantageusement, cet anti-inflammatoire est d'origine naturelle. De préférence, il s'agit de phytostérol de réglisse.

Il est par exemple connu que certaines dyschromies, comme les taches de vieillesse, apparaissent avec l'âge. Les inventeurs ont observé qu'en luttant contre le vieillissement, notamment grâce à des compositions cosmétiques anti-âge, on diminue les manifestations cutanées liées à l'âge et notamment on prévient l'apparition des taches de vieillesse.

Ainsi, suivant un premier mode de réalisation particulier de l'invention, on associe à un actif cosmétique connu pour son action anti-âge, le composé de formule générale (I).

De préférence, l'actif cosmétique anti-âge est un composé organo-silicé biologiquement actif, du type des silanols commercialisés par la Demanderesse.

Avantageusement, ce composé organo-silicé répond à la formule générale (II) suivante : 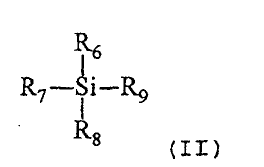 dans laquelle
un au moins des radicaux R₆, R₇, R₈, R₉ représente un groupement hydroxyle ou un groupe hydrolysable comprenant au moins un atome d'oxygène tel qu'il existe une liaison Si-O, et au moins un autre desdits radicaux R₆, R₇, R₈, R₉ représente un groupement alkyle.

De plus, il est démontré que des phénomènes de glycation, de peroxydation des lipides ou des situations de stress oxydatifs peuvent avoir pour conséquence de faire apparaître une pigmentation excessive au niveau cutané. Il est par exemple montré que la synthèse de composés du type lipofuscine, polymère de dérivés d'aldéhydes connu pour être un pigment dû à l'âge, est régulée par des réactions de pro- et d'anti-oxydation. C'est pourquoi, suivant un mode de réalisation particulier, les compositions cosmétiques selon l'invention sont réalisées en associant au moins un composé de formule générale (I) avec au moins un antioxydant.

Par ailleurs, on sait à présent que la destruction d'ADN et/ou sa réparation induit une mélanisation.

Suivant un second mode de réalisation particulier de l'invention, les compositions cosmétiques selon l'invention comprennent, outre le composé de formule (I) et éventuellement mais pas obligatoirement un composé organosilicié, un composé connu notamment pour ses propriétés protectrices et/ou réparatrices de l'ADN.

De préférence, ce composé aux propriétés protectrices de l'ADN est un pseudo-dipeptide du type de ceux obtenus par couplage de l'histamine et d'un acide aminé et décrits dans la demande de brevet internationale W094/19325. De préférence, ce pseudo-dipeptide est la β-alanyl-histamine, également connu pour ses propriétés antioxydantes et pour sa capacité à réduire les hydroperoxydes d'acides gras.

Les proportions de composé de formule générale (I) dans les compositions selon l'invention sont fonction des ingrédients entrant dans ces compositions, et peuvent varier de 0,1 % à 20 % en poids. Toutefois, les proportions préférées varient entre 0,5 % et 5 % en poids.

Dans toutes les compositions cosmétiques selon l'invention, l'excipient peut être tout excipient cosmétiquement acceptable. Avantageusement, l'excipient est une micro-dispersion sans tensio-actif.

Suivant un mode de réalisation particulier de l'invention, les compositions cosmétiques selon l'invention se présentent sous forme de liposomes, de microparticules ou de nanoparticules.

Les compositions cosmétiques selon l'invention peuvent également se présenter sous toute forme cosmétiquement appropriée, et notamment sous toutes les formes appropriées à un usage topique, telle que sous forme de crème, de lait, de lotion ou de gel.

L'invention a également pour objet un agent inhibiteur de la mélanogénèse comprenant une des compositions cosmétiques précédemment décrites.

Les exemples qui suivent illustrent non limitativement l'activité, inhibitrice de la mélanine, des composés de formule (I) entrant dans les compositions selon l'invention.

### Exemple 1 :

On teste l'activité inhibitrice de la mélanine, *in vitro,* de l'acide 1-amino-éthylphosphinique de formule développée (Ia) suivante :

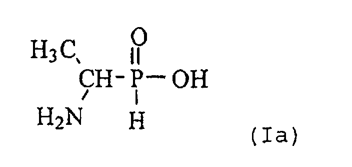

La référence utilisée est l'acide ascorbique en solution à 0, 3. 10⁻³ mole.l⁻¹.
On mélange 2 ml de solution de L-DOPA, 1,25 ml de tyrosinase (2500 Unités/ml), 2 ml du produit (Ia) en solution à 1 10⁻³ mole.l⁻¹, et on complète avec 20 ml de tampon phosphate (NaH₂PO₄ à 0,1 mole.l⁻¹+ Na₂HPO₄ à 0,1 mole.l⁻¹, pH 6,8). On laisse 4 heures à 37°C sous agitation, puis une nuit à température ambiante à l'abri de la lumière. On ajoute 1 ml d'acide chlorhydrique concentré, on centrifuge 5 mn à 4000 tours/minutes. On rince le culot avec 10 ml d'eau distillée, on centrifuge une seconde fois. On fait un reflux sur le culot avec de l'acide concentré HCl 6N pendant 48 heures.

Le culot séché est repris dans 10 ml de toluène, soumis aux ultrasons pendant 2 mn, remis 2 heures à 37 °C puis soumis à nouveau aux ultrasons pendant 5 m. On mesure la densité optique à 400 nm.
Le pourcentage d'inhibition de la mélanine par l'acide 1-amino-éthylphosphinique est de 46 %.

### Exemple 2 :

On teste l'activité inhibitrice de la mélanine, *in vitro,* de l'acide 1-amino-3-méthylbutylphosphinique de formule développée (Ib) suivante : 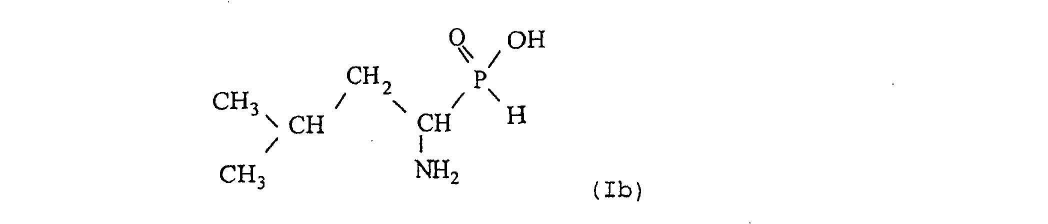 en appliquant le mode opératoire de l'exemple 1.
Le pourcentage d'inhibition de la mélanine par l'acide 1-amino-3-méthylbutylphosphinique est de 44 %.

### Exemple 3 :

On étudie l'activité inhibitrice de la mélanine, de l'acide 1-amino-éthylphosphinique (Ia), sur des cultures de cellules S91 de mélanome.
Le modèle d'étude utilisé a été mis au point par Gilchrest (1987). Les cellules mises en culture sont issues d'un mélanome de Cloudman et répondent, comme des mélanocytes normaux, à une stimulation ultraviolette, par une augmentation de la mélanogénèse. L'évaluation de la mélanogénèse est effectuée à travers le dosage de la mélanine et la recherche de l'activité des enzymes impliquées dans ce métabolisme : la tyrosinase et la dopa-tautomérase. Les cellules S91 sont ensemencées à 2 x 10⁵ cellules dans des boîtes de 60 mm de diamètre et maintenues dans du milieu de Eagle modifié par Dulbecco et supplémenté avec 10 % de sérum de veau foetal. Quand les cellules ont approximativement atteint les 50 % de confluence, les cellules sont irradiées. L'irradiation est de 10 mJ/cm² à la longueur d'onde de 285 ± 5 nm. Durant l'irradiation, le milieu est remplacé par une solution de phosphate salin (PBS) pour éliminer la formation de dérivés toxiques. Immédiatement après l'irradiation, le PBS est remplacé par du milieu frais supplémenté avec 7 % de sérum de veau foetal avec ou sans l'actif. Après 16 heures d'incubation, le milieu est éliminé et remplacé par du milieu normal avec ou sans l'actif. L'évaluation de l'effet est effectuée 24 et 48 heures après l'irradiation. Le témoin de culture non irradié est manipulé de façon identique mais placé à l'obscurité pendant l'irradiation. On dose ensuite la mélanine : les mélanocytes sont trypsinisés, lavés deux fois avec du PBS et solubilisés dans 0,8 ml de NaOH 1N, avec une agitation au vortex vigoureuse pendant 10-15 minutes et un chauffage à 60-80°C. La concentration de la mélanine est déterminée par la mesure de la densité optique à 475 nm..
Le témoin, à savoir l'acide kojique, est utilisé à une concentration de 3,3.10⁻³ mole.l⁻¹ et, à cette concentration, diminue le taux de mélanine dans les cellules de 45,2 %. Le composé (Ia), à une concentration de 0,5.10⁻³ mole.l⁻¹, c'est à dire beaucoup plus faible que celle du témoin, diminue le taux de mélanine dans les cellules de 47,2 %.

### Exemple 4 : Microdispersion huile dans l'eau sans tensio-actif

| | |
|---|---|
| Polyisobutène hydrogéné | 10,75 g |
| huile minérale | 3,00 g |
| heptanoate de stéaryle | 7,25 g |
| parahydroxybenzoate de méthyle sodé | 0,20 g |
| parahydroxybenzoate de propyle | 0,10 g |
| Acide 1-amino-éthylphosphinique | 1,00 g |
| ALISTIN | 0,20 g |
| Carbomer | 0,50 g |
| Eau | 79,00 g |

### Exemple 5 : Gel

| | |
|---|---|
| Carbomer | 0,70 g |
| parahydroxybenzoate de méthyle sodé | 0,20 g |
| parahydroxybenzoate de propyle | 0,10 g |
| Acide 1-amino-éthylphosphinique | 2,00 g |
| Extrait de Mûrier | 3,00 g |
| Eau | 94,00 g |

### Exemple 6 : Crème

| | |
|---|---|
| polyacrylamide | |
| C₁₃₋₁₄ isoparafine | 2,00 g |
| Laureth 7 | |
| imidazolidinyl urée | 0,30 g |
| parahydroxybenzoate de méthyle sodé | 0,10 g |
| parahydroxybenzoate de propyle | 0,05 g |
| Acide 1-amino-éthylphosphinique | 3,00 g |
| glyceryl stéarate | |
| steareth-25 | 10,00 g |
| ceteth-20 | |
| alcool stéarique | |
| Cétéaryl octanoate | 10,00 g |
| huile de macadamia | 10,00 g |
| dimethicone | 0,2 g |
| dimethicone copolyol | 0,3 g |
| glycérine | 3,00 g |
| Eau qsp | 100,00 g |

## Revendications

1. Composition cosmétique utile notamment pour le blanchiment de la peau, **caractérisée en ce qu'**elle contient, en association avec tout excipient approprié au moins un composé de formule générale (I) : 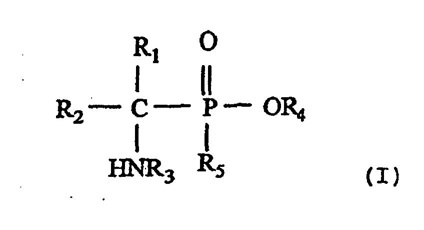 dans laquelle :
R₁ représente un atome d'hydrogène, un groupement (C₁-C₄) alkyle linéaire ou ramifié ou un groupement thiazoline,
R₂, R₃, et R₄ représentent chacun un atome d'hydrogène,
R₅ représente un atome d'hydrogène ou un groupement hydroxyle.

2. Composition cosmétique selon la revendication 1, **caractérisée en ce que** ledit composé de formule (I) est tel que R₁ représente un groupement (C₁-C₄) alkyle, R₂ et R₃ représentent chacun un atome d'hydrogène, et R₅ représente un atome d'hydrogène.

3. Composition cosmétique selon la revendication 1 ou 2, **caractérisée en ce que** ledit composé de formule (I) est l'acide 1-amino-éthylphosphinique.

4. Composition cosmétique selon la revendication 1 ou 2, **caractérisée en ce que** ledit composé de formule (I) est l'acide 1-amino-3-méthylbutylphosphinique.

5. Composition cosmétique selon l'une quelconque des revendications 1 à 4, **caractérisée en ce qu'**elle comprend en outre des anti-inflamcnatoires, d'autres agents de blanchiment de la peau, et/ou des antioxydants.

6. Composition cosmétique selon l'une quelconque des revendications 1 à 5, **caractérisée en ce que** la teneur en composé de formule générale (I) est comprise entre 0,1 et 20 % en poids par rapport au poids total de la composition.

7. Composition cosmétique selon la revendication 6, **caractérisée en ce que** la teneur en composé de formule générale (I) est comprise entre 0,5 et 5 % en poids par rapport au poids total de la composition.

8. Composition cosmétique selon l'une quelconque des revendications 1 à 7, **caractérisée en ce qu'**elle comprend en outre un composé organo-silicé de formule générale (II) suivante : 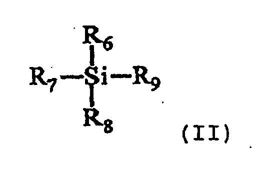 dans laquelle un au moins des radicaux R₆, R₇, R₈ ou R₉ représente un groupement hydroxyle ou un groupement alcoxy, acyloxy ou aryloxy, amine ou un atome d'halogène et au moins un autre desdits radicaux R₆, R₇, R₈ ou R₉ représente un groupement alkyle.

9. Composition cosmétique selon l'une quelconque des revendications 1 à 8, **caractérisée en ce qu'**elle comprend en outre un pseudo-dipeptide du type de ceux obtenus par couplage de l'histamine et d'un acide aminé.

10. Composition cosmétique selon la revendication 9, **caractérisée en ce que** ledit pseudo-dipeptide est la β-alanyl-histamine.

11. Composition cosmétique selon l'une quelconque des revendications 1 à 10, **caractérisé en ce que** ledit excipient est une micro-dispersion sans tensio-actif.

12. Composition cosmétique selon l'une quelconque des revendications 1 à 10, **caractérisé en ce qu'**elle se présente sous forme de liposomes, de microparticules ou de nanoparticules.

13. Composition cosmétique selon l'une quelconque des revendications 1 à 12, **caractérisée en ce qu'**elle se présente sous la forme d'une crème, d'un lait, d'une lotion, ou d'un gel.

14. Agent inhibiteur de la mélanogénèse, **caractérisé en ce qu'**il comprend une composition cosmétique selon l'une quelconque des revendications 1 à 13.

## Patentansprüche

1. Kosmetische Zusammensetzung, die insbesondere zum Bleichen der Haut verwendbar wird, **dadurch gekennzeichnet, dass** sie in Zusammenhang mit jeder geeigneten Grundmasse mindestens eine Verbindung der folgenden allgemeinen Formel (I) enthält: 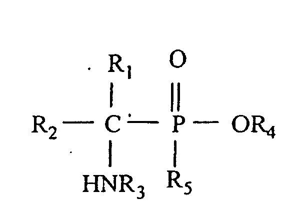 wobei:
R₁ ein Wasserstoffatom, eine geradkettige oder verzweigte Alkylgruppe (C₁-C₄) oder eine Thiazolingruppe darstellt,
R₂, R₃ und R₄ jeweils ein Wasserstoffatom darstellen,
R₅ ein Wasserstoffatom oder eine Hydroxylgruppe darstellt.

2. Kosmetische Zusammensetzung gemäß Anspruch 1, **dadurch gekennzeichnet, dass** die Verbindung der Formel (I) derart ist, dass R₁ eine Alkylgruppe (C₁-C₄) darstellt, R₂ und R₃ jeweils ein Wasserstoffatom darstellen und R₅ ein Wasserstoffatom darstellt.

3. Kosmetische Zusammensetzung gemäß Anspruch 1 oder 2,
**dadurch gekennzeichnet, dass** die Verbindung der Formel (I) eine 1-Amino-ethylphosphinsäure ist.

4. Kosmetische Zusammensetzung gemäß Anspruch 1 oder 2,
**dadurch gekennzeichnet, dass** die Verbindung der Formel (I) eine 1-Amino-3-methylbutylphosphinsäure ist.

5. Kosmetische Zusammensetzung gemäß einem der Ansprüche 1 bis 4, **dadurch gekennzeichnet, dass** sie ferner entzündungshemmende Mittel, andere Agenzien zum Bleichen der Haut und/oder Antioxidationsmittel umfasst.

6. Kosmetische Zusammensetzung gemäß einem der Ansprüche 1 bis 5, **dadurch gekennzeichnet, dass** der Gehalt an der Verbindung der allgemeinen Formel (I) zwischen 0,1 und 20 Gew.-% des Gesamtgewichts der Zusammensetzung liegt.

7. Kosmetische Zusammensetzung gemäß Anspruch 6, **dadurch gekennzeichnet, dass** der Gehalt an der Verbindung der allgemeinen Formel (I) zwischen 0,5 und 5 Gew.-% des Gesamtgewichts der Zusammensetzung liegt.

8. Kosmetische Zusammensetzung gemäß einem der Ansprüche 1 bis 7, **dadurch gekennzeichnet, dass** sie ferner eine Organosiliziumverbindung der folgenden allgemeinen Formel (II) umfasst: 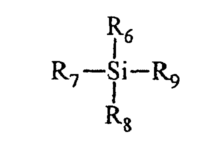 wobei mindestens eines der Radikale R₆, R₇, R₈ oder R₉ eine Hydroxylgruppe oder eine Alkoxy-, Acyloxy- oder Aryloxy-, Amingruppe oder ein Halogenatom darstellt und mindestens ein anderes der Radikale R₆, R₇, R₈ oder R₉ eine Alkylgruppe darstellt.

9. Kosmetische Zusammensetzung gemäß einem der Ansprüche 1 bis 8, **dadurch gekennzeichnet, dass** sie ferner ein Pseudo-Dipeptid der Art derjenigen, die durch Koppeln des Histamins mit einer Aminosäure erhalten werden, umfasst.

10. Kosmetische Zusammensetzung gemäß Anspruch 9, **dadurch gekennzeichnet, dass** das Pseudo-Dipeptid das β-Alanyl-histamin ist.

11. Kosmetische Zusammensetzung gemäß einem der Ansprüche 1 bis 10, **dadurch gekennzeichnet, dass** die Grundmasse eine Mikrodispersion ohne ein grenzflächenaktives Mittel ist.

12. Kosmetische Zusammensetzung gemäß einem der Ansprüche 1 bis 10, **dadurch gekennzeichnet, dass** sie in Form von Liposomen, Mikropartikeln oder Nanopartikeln vorliegt.

13. Kosmetische Zusammensetzung gemäß einem der Ansprüche 1 bis 12, **dadurch gekennzeichnet, dass** sie in Form einer Creme, einer Milch, einer Lotion oder eines Gels vorliegt.

14. Ein Agens zum Hemmen der Melanogenese, **dadurch gekennzeichnet, dass** es eine kosmetische Zusammensetzung gemäß einem der Ansprüche 1 bis 13 umfasst.

## Claims

1. Cosmetic composition useful notably in the whitening of skin **characterized in that** it contains, in association with any suitable excipient, at least one compound of the following general formula (I) : 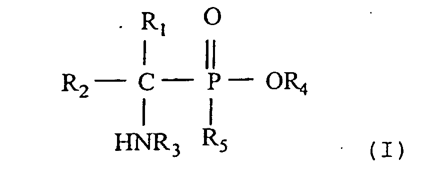 wherein :
R₁ represents a hydrogen atom, a linear or branched alkyl group (C1-C4) or a thiazoline group,
R₂, R₃ and R₄ each represent a hydrogen atom,
R₅ represents a hydrogen atom or a hydroxyl group.

2. Cosmetic composition according to claim 1 **characterized in that** said compound of formula (I) is such as R₁ represents a (C₁-C₄) alkyl group, R₂ or R₃ represent each one a hydrogen atom, and R₅ represents a hydrogen atom

3. Cosmetic composition according to claim 1 or 2 **characterized in that** said compound of formula (I) is the 1-amino-ethylphosphinic acid.

4. Cosmetic composition according to claim 1 or 2 **characterized in that** said compound of formula (I) is the 1-amino-3-methylbutylphosphinic acid.

5. Cosmetic compound according to any of the claim 1 to 4, **characterized in that** it contains in addition antiinflammatory agents, other skin whitening agents, and/or an antioxidant.

6. Cosmetic compound according to any of the claim 1 to 5, **characterized in that** the content of the compound of general formula (I) is included between 0,1 and 20% in weight in reference to the total weight of the composition.

7. Cosmetic composition according to the claim 6, **characterized in that** the content of the compound of general formula (I) is included between 0,5 and 5% in weight in reference to the total weight of the composition.

8. Cosmetic composition according to any of the claim 1 to 7, **characterized in that** it contains in addition an organosilicon compound of the following general formula (II) : 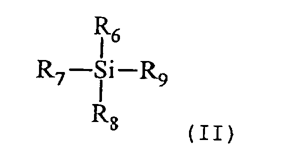 wherein at least one of the radicals R₆, R₇, R₈ or R₉ represents a hydroxyl group or an alcoxy, acyloxy, aryloxy or amine group, or a halogen atom and at least an other of said radicals R₆, R₇, R₈ or R₉ represents an alkyl group.

9. Cosmetic composition according to any of the claim 1 to 8, **characterized in that** it contains in addition a pseudo-dipeptide such as the type obtained by the coupling between histamine and an aminoacid.

10. Cosmetic composition according to the claim 9, **characterized in that** said pseudo-dipeptide is the β-alanyl-histamine.

11. Cosmetic composition according to any of the claim 1 to 10, **characterized in that** said excipient is a micro-dispersion without surfactant.

12. Cosmetic composition according to any of the claim 1 to 10, **characterized in that** it is in the form of liposomes, microparticles or nanoparticles.

13. Cosmetic composition according to any of the claim 1 to 12, **characterized in that** it is in the form of a cream, a milk, a lotion or a gel.

14. Melanogenesis inhibiting agent **characterized in that** it includes a cosmetic composition according to any of the claim 1 to 13.
